# EUROPEAN PATENT APPLICATION

(11) **EP 1 829 561 A1**
(43) Date of publication of application: **05.09.2007**
(21) Application number: 06004353.6
(22) Date of filing: 03.03.2006
(51) Int. Cl.: A61L 15/22, A61L 15/42

(54) **Thermoplastic absorbent materials for proteinaceous or serous bodily fluids**

(71) Applicant: The Procter and Gamble Company, Cincinnati, Ohio 45202 (US)
(72) Inventor: Gonzales, Denis, Alfred, 1150 Woluve St Pier (BE); Macbeath, Calum, 65023 Francavilla al Mare ( Chieti ) (IT); Lunetto, Pietro, 65025 Manoppello ( Pescara ) (IT)
(74) Representative: Hampton, Matthew John

(57) **Abstract**

The present invention relates to a thermoplastic absorbent material for the absorption of proteinaceous or serous body fluids, particularly menses. The thermoplastic absorbent material has a better handling towards such fluids, both in terms of retention capacity and absorption rate, comprising a thermoplastic base material and a superabsorbent material in particle form dispersed therein.

## Description

### Field of the Invention

The present invention relates to thermoplastic absorbent materials for absorption of proteinaceous or serous body fluids. The materials comprise a thermoplastic polymeric base material having dispersed therein particles of a superabsorbent material, and have an improved capacity of acquiring and retaining such fluids. Particularly the thermoplastic absorbent materials of the present invention can be used in absorbent articles such as sanitary napkins, wherein the body fluid is menses, as well as tampons, interlabial devices, panty liners, wound dressings, breast pads or the like.

### Background of the Invention

In general the absorption and retention of body fluids such as urine, menses, etc., are accomplished by use of absorbent articles containing absorbent materials. Such articles generally include disposable diapers, incontinence pads, sanitary napkins, tampons, wound dressings, nursing pads, and the like. Generally, the most used absorbent materials are cellulose materials (preferably, defiberised wood pulp) and superabsorbent materials. In particular, when generally referring to disposable diapers or sanitary napkins and the like presently available in the market, the cellulose materials are in the form of bat or sheet, typically further containing particulate absorbent materials, usually referred to in the art as superabsorbents or hydrogelling materials, which allow to manufacture thin but very absorbent core structures. A common need when incorporating particulate superabsorbent material in a core structure is to stabilize it within the structure in order to prevent it from displacing or from spilling outside, with obvious drawbacks in terms of effectiveness, user friendliness, and ultimately acceptance of the product by the consumer. Supported or unsupported films made entirely from superabsorbent polymers have been suggested as absorbent core for disposable absorbent articles but they are generally stiff and frequently break apart especially when in dry state. In addition to these solutions, in the art there are different suggestions of absorbent materials comprising a particulate superabsorbent material dispersed within a matrix of a thermoplastic base material. The composite absorbent thermoplastic material can generally be incorporated in an absorbent structure by melting it and by applying onto a selected substrate in any desired pattern or configuration, wherein upon cooling and solidification the material is stably positioned by typically adhering to the substrate and does not move or displace into the structure. Also, the presence of free flowing particles in the structure and related drawbacks are avoided. For example, US 4318408 describes an aqueous-fluid-absorbent non-disintegrative product which comprises a water-insoluble substantially non-swelling matrix of an elastomeric polymer having at least partially embedded therein particles of a water-insoluble but water-swellable organic polymeric absorbent material. Similarly, EP 1013291 describes a hot melt adhesive containing a superabsorbent polymer. WO 99/57201 illustrates compositions comprising a thermoplastic component and a superabsorbent polymer, said compositions in form of a film layer or applied to a disposable absorbent article with various hot melt adhesive application techniques. WO 03/049777, assigned to The Procter & Gamble Company, discloses an improved liquid absorbing thermoplastic material comprising a thermoplastic base material with particles of superabsorbent material dispersed therein, wherein the thermoplastic base material itself has a preferred composition with inherent liquid absorption capacity. The material is particularly effective in liquid acquisition and handling, and moreover has an increased cohesiveness when wet.

A typical and more specific use of thermoplastic absorbent materials comprising a thermoplastic base material with particles of superabsorbent material dispersed therein is in absorbent articles for absorption of proteinaceous or serous body fluids such as menses, blood, plasma, vaginal secretions, mucus or milk. Said articles are well known in the art, and typically comprise feminine hygiene articles such as sanitary napkins, panty liners, tampons, and interlabial devices, as well as wound dressings, breast pads or the like, usually having an absorbent structure.

A thermoplastic absorbent material can be beneficially provided in a stable and effective configuration/pattern in said articles, typically in the absorbent core, in order to increase their absorption and retention characteristics. Conventional superabsorbent materials known in the art for use in absorbent articles typically comprise water-insoluble, water-swellable, hydrogel-forming crosslinked absorbent polymers which are capable of absorbing large quantities of liquids and of retaining such absorbed liquids under moderate pressure. In general, known thermoplastic absorbent materials comprising conventional absorbent gelling materials commonly have good absorption and retention characteristics to water and urine; however, there still remains room for improvement for absorption and retention towards certain liquids. In particular, proteinaceous or serous body fluids such as typically menses, blood, plasma, vaginal secretions, mucus or milk, are particularly difficult to be effectively absorbed and retained into thermoplastic absorbent materials containing superabsorbent materials since said materials do not show enough absorption and retention characteristics towards said proteinaceous or serous body fluids.

Such not optimal absorption and retention are mainly caused by poor permeability of known thermoplastic absorbent materials comprising superabsorbent materials towards such proteinaceous or serous body fluids, in turn due to the viscosity and/or to the complex nature of the fluids. For example, plasma, blood and menses components, including red cells, white cells, soluble proteins, cellular debris and/or mucus, slow down the absorption of these fluids by superabsorbents. Because these fluids comprise many complex components, and are often typically rather thick, absorption into known thermoplastic absorbent materials comprising superabsorbent polymers is difficult. This translates into a slower initial uptake rate of the fluid into the superabsorbent material, and in turn in the thermoplastic absorbent material, which can result in a lower final absorption and retention capacity if gel blocking occurs before the superabsorbent material is fully swollen.

Attempts to increase the absorption and retention capacity of superabsorbent materials as such for proteinaceous or serous fluids, such as blood or menses, have led for example to chemical modification of these superabsorbent materials, such as by differential crosslinking between surface and bulk of the particle, or treatment with additives, for example to improve wettability with blood by surface treatment of particulate absorbent materials using particular compounds, as disclosed in US Patent 4,190,563. Alternatively, or in combination, morphological modifications of the superabsorbent materials are also known in the art, for example adopting preferred shapes or dimensions for the particles.

However, although such known approaches have achieved some success in absorption and retention of proteinaceous or serous body fluids by said modified superabsorbent materials, they are associated to several undesirable processing and consumer use concerns, which ultimately transfer to thermoplastic absorbent materials incorporating such superabsorbent materials in particle form. Provision of chemically and/or morphologically modified superabsorbent materials certainly adds complexity, and cost, to the production process for the manufacture of absorbent articles for absorption of proteinaceous or serous body fluids. Moreover, chemically modified superabsorbent materials can loose effectiveness during use, for example a surface coated additive can be washed away from the superabsorbent material by succeeding applications of fluid.

Consequently, there remains a need for further improvements in thermoplastic absorbent materials for absorption of proteinaceous or serous body fluids, such as for example when used in sanitary napkins, which have increased fluid absorption and retention capacity, particularly a high intake rate for such body fluids. Additionally, it would be beneficial if a reduced amount of thermoplastic absorbent material as compared to similar known materials could be used to achieve said results.

### Summary of the invention

It has been surprisingly discovered that the objectives above can be achieved by a thermoplastic absorbent material comprising a thermoplastic base material and particles of a superabsorbent material dispersed therein, wherein the particles have an irregular shape and an average particle size in dry state of from 0.1 µm and 50 µm, or from 1 µm and 30 µm, or preferably from 1 µm and 20 µm, and wherein the thermoplastic absorbent material has an Elastic Modulus G' of from 50 Pa to 100.000 Pa, or from 75 and 50.000 Pa, or from 75 and 10.000 Pa, or preferably from 100 and 1.000 Pa, measured at 38°C and 1 rad⁻¹.

The thermoplastic absorbent materials of the present invention are particularly suitable for absorption of proteinaceous or serous body fluids.

### Detailed Description of the Invention

The term "absorbent article" is used herein as including any article able to receive and/or absorb and/or contain and/or retain proteinaceous or serous body fluids. "Absorbent articles" as referred to herein include, without being limited to, feminine hygiene articles such as sanitary napkins, panty liners, tampons, interlabial devices, as well as wound dressings, breast pads and the like. Particularly, the disposable absorbent article is described below by reference to a sanitary napkin.

The term "disposable" is used herein to describe articles that are not intended to be laundered or otherwise restored or reused as an article (i.e., they are intended to be discarded after a single use and, preferably, to be recycled, composted or otherwise disposed of in an environmentally compatible manner).

As used herein, the term 'wearer-facing', or alternatively 'body-facing', surface refers to the surface of the component of the article generally oriented to face the wearer skin and/or mucosal surface during use of the article. As used herein, the term 'garment facing' surface refers to the opposite outer surface of the article, typically the surface directly facing the garment of a wearer, if worn in direct contact with a garment.

As used herein, the term 'proteinaceous or serous body fluids' refers to bodily fluids secreted by the body which comprise several complex components such as for example red cells, white cells, soluble proteins, cellular debris, and/or mucus, and typically have a viscosity higher than urine or water. Some proteinaceous or serous body fluids include for example menses, blood, plasma, vaginal secretions, or also mucus or milk.

The term 'thermoplastic' as used herein refers to the ability of materials to soften and possibly even melt at raised temperatures and to harden again at reduced temperatures, while having substantially the same material characteristics after the hardening step as before the softening and/or melting step.

In the following, non-limiting embodiment of the present invention, a sanitary napkin is described as an exemplary absorbent article comprising the thermoplastic absorbent material of the present invention, typically comprising as main elements: a topsheet, facing the user of the article during use and being liquid pervious in order to allow liquids, particularly body fluids, to pass into the article; a backsheet, providing liquid containment such that absorbed liquid does not leak through the article, this backsheet conventionally providing the garment facing surface of the article; and an absorbent core comprised between the topsheet and the backsheet and providing the absorbent capacity of the article to acquire and retain liquid which has entered the article through the topsheet. Of course, other types of absorbent articles comprising the thermoplastic absorbent material of the present invention may not comprise one or more of the above elements, such as for example tampons or wound dressings which typically do not have a backsheet, as can be readily determined by the man skilled in the art. All absorbent articles however have an absorbent core or element, typically comprising the thermoplastic absorbent material of the present invention, which is any absorbent means provided in the article and which is capable of absorbing and retaining proteinaceous or serous body fluids, such as for example menses.

The elements constituting the absorbent article comprising the thermoplastic absorbent material of the present invention may be conventional, as it is known in the art and described hereinafter with reference to a sanitary napkin.

### Topsheet.

The topsheet is compliant, soft feeling, and non-irritating to the wearer's skin. The topsheet also can be elastically stretchable in one or two directions. Further, the topsheet is liquid pervious permitting body fluids to readily penetrate through its thickness.

A suitable topsheet can be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims.

### Backsheet.

The backsheet prevents the liquids absorbed and contained in the absorbent element from wetting articles that contact the absorbent article such as pants, pajamas and undergarments. The backsheet is typically impervious to liquids like body fluids and can be manufactured from a thin plastic film, although other flexible liquid impervious materials can also be used. The backsheet can also be optionally breathable, thus permitting the transfer of water vapor and preferably both water vapor and air through it and thus allows reduction of humid and occlusive environment on the skin contacted with the article.

### Absorbent core.

The absorbent core, which is disposed between the topsheet and the backsheet, absorbs and retains bodily fluids that have penetrated the topsheet after discharge by a wearer. The absorbent core may be any absorbent means which is capable of absorbing or retaining bodily liquids (typically menses for a sanitary napkin). The absorbent core may be manufactured in a wide variety of sizes and shapes (e.g., rectangular, oval, hourglass, dog bone, asymmetric, etc.) and from a wide variety of liquid-absorbent materials commonly used in sanitary napkins and other absorbent articles such as comminuted wood pulp which is generally referred to as airfelt. Examples of other suitable absorbent materials include creped cellulose wadding; meltblown polymers including coform; chemically stiffened, modified or cross-linked cellulosic fibers; synthetic fibers such as crimped polyester fibers; peat moss; tissue including tissue wraps and tissue laminates; absorbent foams; absorbent sponges. Typically, the thermoplastic absorbent material of the present invention can be comprised in the absorbent core of an absorbent article.

### Thermoplastic absorbent material.

The thermoplastic absorbent material of the present invention comprises a thermoplastic polymeric base material, or, more simply, a thermoplastic base material, and particles of a superabsorbent material dispersed therein. Typically, the thermoplastic absorbent material of the present invention comprises from 20% to 99%, or from 30% to 90%, or preferably from 40% to 70% by weight of the total composition of a thermoplastic polymeric base material and from 1% to 80%, or from 10% to 70%, or preferably from 30% to 60%, by weight of the total composition of particles of superabsorbent material.

According to the present invention, any superabsorbent material known to the skilled person and used in absorbent articles, such as feminine care absorbent articles (e.g. sanitary napkins, panty liners or incontinence articles) or baby care absorbent articles (e.g. diapers) can be used herein. The particles of superabsorbent material for use herein are characterized by their irregular shape and their average particle size in dry state of from 0.1 µm to 50 µm. The thermoplastic absorbent material of the present invention also has a selected rheology with an Elastic Modulus G' of from 50 Pa to 100.000 Pa.

"Particles" as used herein refers to discrete flakes, granules, powder and the like or mixtures thereof, of a certain material, in particular superabsorbent material. The term "particles" herein may also comprise agglomerations or aggregations of discrete flakes, granules, powder and the like of a certain material.

For purposes of the present invention, particle size is defined as the dimension of a particle which is determined by means of any suitable method known in the art for particle sizes comprised in the range according to the present invention. Particularly indicated are laser light scattering analysis or laser diffraction analysis. The average particle size of a given sample is defined as the particle size corresponding to a cumulative distribution of 50% of the particles of the sample. In other words, the average particle size of a given sample of superabsorbent material particles is defined as the particle size which divides the sample in half on a mass basis, i.e., half of the sample by weight will have a particle size greater than the average particle size and half of the sample by weight will have a particle size less than the particle size.

The term "irregular shape" as used herein refers to the shape of particles of superabsorbent material which are neither smooth nor rounded, but have sharp angles and edges in their shapes and on their surfaces. Typically, particles of superabsorbent material having an irregular shape according to the present invention can be obtainable, and are typically made, by suitably grinding with known means larger particles in order to obtain finer particles with the desired selected average particle size as described above.

The term "in dry state", as used herein, refers to the state of a superabsorbent material in absence of any water or water based fluid, and more in general, of any fluid which interacts with the polyacrylate based material by being absorbed and causing swelling thereof. It can hence encompass the superabsorbent material in presence of a non aqueous solvent which is not absorbed, and does not cause swelling of the material.

The average particle size of the particles of superabsorbent material for use herein is in dry state from 0.1 µm and 50 µm, or from 1 µm and 30 µm, or preferably from 1 µm and 20 µm.

In an embodiment of the present invention, it may be desirable the particles of superabsorbent material comprised in the thermoplastic absorbent material also have an average surface area per volume of at least 0.50 m²/cm³, or at least 0.60 m²/cm³, or preferably at least 0.70 m²/cm³. The average surface area of the particles is evaluated with any suitable means known in the art, for example laser light scattering analysis or laser diffraction analysis used for the determination of the average particle size as mentioned above are also suitable for the average surface area measurement.

The thermoplastic absorbent material of the present invention has an Elastic Modulus G' of from 50 to 100.000 Pa, or of from 75 and 50.000 Pa, or of from 75 and 10.000 Pa, or preferably of from 100 and 1.000 Pa, measured at a temperature of 38°C and a frequency of 1 rad⁻¹.

It may be desirable the thermoplastic absorbent material also has a Tan δ of at least 0.15, or at least 0.30, or preferably a least 0.70, measured at a temperature of 38°C and a frequency of 1 rad⁻¹.

These conditions of temperature and frequency are representative of the actual usage conditions of the thermoplastic absorbent material of the present invention, typically comprised within an absorbent article for absorption of proteinaceous or serous body fluids, such as for example a sanitary napkin for absorption of menses, worn in direct contact with the body. The reference temperature of 38°C in fact substantially corresponds to the body temperature, and the low frequency can be considered to represent the stresses typically applied to the thermoplastic absorbent composition during normal use of a typical absorbent article.

This rheological behaviour of the thermoplastic absorbent material of the present invention corresponds to a certain "softness" of the material in usage conditions, which improves the acquisition and handling of the fluids which come in contact with the thermoplastic absorbent material of the present invention in order to be absorbed. In addition, said thermoplastic absorbent materials provide an advantage also at process conditions, i.e. typically in the molten state, in that they can be still typically processable as a hot melt, as will be explained more in detail further on.

Without being bound by any theory, it is believed that the irregular shape of the particles of superabsorbent material, in combination with the selected low or very low average particle size, optionally in combination with the selected average surface area of the particles, and within a thermoplastic absorbent material having an overall relative softness in usage conditions, provide a thermoplastic absorbent material which is particularly effective in the acquisition and absorption of proteinaceous or serous body fluids. Said complex fluids in fact can be rapidly acquired and absorbed by the thermoplastic absorbent material since on the one hand the small average particle size and the irregular shape of the particles of superabsorbent material, optionally in combination with a selected high average surface area, maximize the surface of contact between the superabsorbent material and the complex fluid, and hence facilitates their interaction and consequently the absorption mechanism. On the other hand, the "softness" of the thermoplastic base material in combination improves acquisition and handling of complex proteinaceous or serous body fluids.

### Thermoplastic polymeric base material.

Any thermoplastic polymeric base material known to the skilled person and used in the construction of absorbent articles, such as feminine care absorbent articles (e.g. sanitary napkins or panty liners) can be used herein.

The thermoplastic base materials for use herein comprise from 5% to 99%, or from 10% to 90%, or from 30% to 70%, or preferably from 40% to 60% by weight of a thermoplastic polymer or a mixture of thermoplastic polymers as an essential element. A variety of different thermoplastic polymers are suitable for use herein. Exemplary thermoplastic polymers for use with the present invention may be block copolymers, amorphous and crystalline polyolefins including homogeneous and substantially linear ethylene/alpha-olefin interpolymers, interpolymers of ethylene such as ethylene-vinylacetate (EVA), ethylene-methyl-acrylate (EMA) and ethylene n-butyl acrylate (EnBa) and mixtures thereof

A wide variety of 'block copolymers' may be useful in the present invention. The group of block copolymers can include linear copolymers of the triblock A-B-A or the diblock A-B type, or radial co-polymer structures having the formula (A-B)x- The A blocks may be non-elastic polymer blocks, typically polyvinylarene blocks, the B blocks may be unsaturated conjugated dienes, such as poly(monoalkenyl) blocks, or hydrogenated versions thereof, x denotes the number of polymeric arms, and x is an integer greater than or equal to one. Suitable block Polyvinylarenes include, but are not limited to, polystyrene, polyalpha-methylstyrene, polyvinyltoluene, and combinations thereof Suitable block B poly(monoalkenyl) blocks include, but are not limited to conjugated diene elastomers such as for example polybutadiene or polyisoprene or hydrogenated elastomers such as ethylene butylene or ethylene propylene or polyisobutylene, or combinations thereof Commercial examples of these types of block copolymers include Europrene^{™} Sol T from EniChem, Kraton^{™} elastomers from Shell Chemical Company, Vector^{™} elastomers from Dexco, Solprene^{™} from Enichem Elastomers and Stereon^{™} from Firestone Tire & Rubber Co.

Amorphous polyolefins or amorphous polyalphaolefins (APAO) are homopolymers, copolymers, and terpolymers of C2-C8 alphaolefins. These materials are typically polymerised by means of processes, which employ Ziegler-Natta catalysts resulting in a relatively broad molecular weight distribution. Commercially available amorphous polyalphaolefins include Rextac^{™} and REXFlex^{™} propylene based homopolymers, ethylene-propylene copolymers and butene-propylene copolymers available from Rexene (Dallas, TX) as well as Vestoplast alpha-olefin copolymers available from Huls (Piscataway, NJ).

Metallocene polyolefins are homogeneous linear and substantially linear ethylene polymers prepared using single-site or metallocene catalysts. Homogeneous ethylene polymers are characterized as having a narrow molecular weight distribution and a uniform short-chain branching distribution. In the case of substantially linear ethylene polymers, such homogeneous ethylene polymers are further characterized as having long chain branching. Substantially linear ethylene polymers are commercially available from The Dow Chemical Company as Affinity^{™} polyolefin plastomers, which are produced using Dow's Insite^{™} technology, whereas homogeneous linear ethylene polymers are available from Exxon Chemical Company under the tradename Exact^{™}. Homogeneous linear and substantially linear ethylene polymers having a relatively low density, ranging from about 0.855 to about 0.885, and a relatively low melt index, for example less than about 50 g/10min are most preferred, particularly for creating elastomeric fibers, films and adhesive compositions that swell upon exposure to water.

The term 'interpolymer' is used herein to indicate a copolymer, terpolymer, or higher order polymer. That is, at least one other comonomer is polymerized with ethylene to make the interpolymer. Interpolymers of ethylene are those polymers having at least one comonomer selected from the group consisting of vinyl esters of a saturated carboxylic acid wherein the acid moiety has up to 4 carbon atoms, unsaturated mono- or dicarboxylic acids of 3 to 5 carbon atoms, a salt of the unsaturated acid, esters of the unsaturated acid derived from an alcohol having 1 to 8 carbon atoms, and mixtures thereof.

If employed uncompounded, the ethylene to unsaturated carboxylic comonomer weight ratio may be desirably greater than about 3:1, or preferably about 2:1. Hence, the comonomer concentration is desirably greater than 30 wt-%, or greater than 33 wt-% or preferably greater than 35 wt-%, with respect to the total weight of the ethylene interpolymer. The melt index of the interpolymers of ethylene may range from about 50 to about 2000, or from about 100 to 1500, or from about 200 to 1200, or preferably from about 400 to 1200 g/10 min. When employing a polymer having too low of a melt index uncompounded, the strength of the polymer tends to constrain the swelling of the particles of polyacrylate based material.

Suitable ethylene/unsaturated carboxylic acid, salt and ester interpolymers include ethylene/vinyl acetate (EVA) ethylene/acrylic acid (EEA) and its ionomers; ethylene/methacrylic acid and its ionomers; ethylene/methyl acrylate (EMA); ethylene/ethyl acrylate; ethylene/n-butyl acrylate (EnBA); as well as various derivatives thereof that incorporate two or more comonomers.

Other suitable thermoplastic polymers that may be employed include polylactide, caprolactone polymers, and poly (hydroxy-butyrate/hydroxyvalerate), certain polyvinyl alcohols, biodegradable copolyesters such as Eastman Copolyester 14766 (Eastman), linear saturated polyesters such as Dynapol or Dynacoll polymers from Huls, poly (ethylene oxide) polyether amide and polyester ether block copolymers available from Atochem (Pebax^{™}, e.g. Pebax MV 3000) or Hoechst Celanese (Rite-flex™) respectively, and polyamide polymers such as those available from Union Camp (Unirez^{™}) or Huls (Vestamelt^{™}) or EMS-Chemie (Griltex^{™}). Other particularly preferred suitable thermoplastic polymers are e.g. polyurethanes, poly-ether-amides block copolymers, polyethylene-acrylic acid and polyethylene-methacrylic acid copolymers, polyethylene oxide and its copolymers, ethylene acrylic esters and ethylene methacrylic esters copolymers, polylactide and copolymers, polyamides, polyesters and copolyesters, polyester block copolymers, sulfonated polyesters, poly-ether-ester block copolymers, poly-ether-ester-amide block copolymers, ionomers, polyethylene-vinyl acetate, polyvinyl alcohol and its copolymers, polyvinyl ethers and their copolymers, poly-2-ethyl-oxazoline and derivatives, polyvinyl pyrrolidone and its copolymers, thermoplastic cellulose derivatives, poly-caprolactone and copolymers, poly glycolide, polyglycolic acid and copolymers, polylactic acid and copolymers, polyureas, polyethylene, polypropylene, or mixtures thereof

Suitable polymers that can be used in the present invention may be thermoplastic polyurethanes, polyesters, ethylene-vinyl acetate copolymers, ethylene-methyl acrylate copolymers, and polyolefins.

Particularly suitable thermoplastic polymers may be selected from thermoplastic poly-ether-amide block copolymers (e.g. Pebax^{™}, thermoplastic poly-ether-ester-amide block copolymers, thermoplastic polyester block copolymers (e.g. Hytrel^{™}, e.g. Hytrel 8171), thermoplastic polyurethanes (e.g. Estane™), or mixtures thereof.

The thermoplastic polymeric base materials for use herein furthermore can comprise from 5% to 90%, or from 10% to 85%, or from 15% to 70%, or preferably from 30% to 65% by weight of a suitable compatible plasticiser or a blend of suitable compatible plasticizers. Suitable 'plasticizers' for use in the present invention generally can include any conventional plasticizers which decrease hardness and modulus, enhance pressure sensitive tack and reduce melt and solution viscosity. It may be desirable that the plasticizer be water soluble or water dispersible or alternatively be a wax-like substance such as polyethylene or polypropylene glycol, glycerin, glycerol and its esters, butylene glycol or sorbitol. Other plasticizers suitable for use in the present invention are esters of sucrose; phthalate plasticizers such as dioctyl phthalate and butyl benzyl phthalate (e. g., Santicizer 160 from Monsanto); benzoate plasticizers such as 1,4- cyclohexane dimethanol dibenzoate (e.g., Benzoflez 352 from Velsicol), diethylene glycol/dipropylene glycol dibenzoate (e.g., Benzoflez 50 from Velsicol), and diethylene glycol dibenzoate where the mole fraction of hydroxyl groups which have been esterified ranges from 0.5 to 0.95 (e.g., Benzoflex 2-45 High Hydroxyl also from Velsicol); phosphite plasticizers such as t-butyl diphenyl phosphate (e.g., Santicizer 154 from Monsanto); adipates; sebacates; epoxidized vegetal oils; polymerised vegetal oils; polyols; phthalates; liquid polyesters such as Dynacol 720 from Huls; glycolates; p-toluene sulfonamide and derivatives; glycols and polyglycols and their derivatives; sorbitan esters; phosphates; monocarboxylic fatty acids (C8-C22) and their derivatives; liquid rosin derivatives having Ring and Ball hydrocarbon oils which are low in aromatic content and which are paraffinic or naphthenic in character and mixtures thereof Plasticizer oils are preferably low in volatility, transparent and have as little color and odor as possible. An example of a preferred plasticizer is Carbowax^{™} polyethylene glycol from Union Carbide. Other preferred plasticizers are PEG 400 and PEG 1500 from Aldrich. The use of plasticizers in this invention also contemplates the use of olefin oligomers, low molecular weight polymers, vegetable oils and their derivatives and similar plasticizing liquids.

According to an embodiment of the present invention suitable plasticizers to be used herein may be hydrophilic plasticizers such as acids, esters, amides, alcohols, polyalcohols, or mixtures thereof among which particularly suitable may be citric acid esters, tartaric acid esters, glycerol and its esters, sorbitol, glycolates, and mixtures thereof, as disclosed in our application WO 99/64505. Said hydrophilic plasticizers have a particularly high polar character and provide the further advantage that they do not impair, and possibly can even enhance, the moisture vapour permeability of the resulting layer or film formed from the thermoplastic polymeric base material and thus the thermoplastic absorbent material of the present invention comprising said hydrophilic plasticizer or blend of plasticizers, when compared to a corresponding film or layer formed from an thermoplastic absorbent material comprising the same components, but without the plasticizer or plasticizers.

These hydrophilic plasticizers or blends of hydrophilic plasticizers can of course also adjust the viscosity of the thermoplastic polymeric base material and thus the thermoplastic absorbent material according to the present invention. Thus, the viscosity can be fine-tuned to the preferred values disclosed infra in order to make said composition processable.

Plasticizers selected among those described in European patent application EP 1,193,289 can also be used herein. Said plasticizers can be selected from the group consisting of esters of phosphoric acid; esters of benzoic, phthalic and trimellitic acids; esters of polycarboxylic oxy-acids; sulphonamides and their derivatives such as sulphonamide-formaldehyde resins; sulfones; esters of poly-valent alcohols; lactides; glycolides; lactones; lactams. Said plasticizers are capable of also providing the thermoplastic polymeric base material and thus the thermoplastic absorbent material with a certain degree of tackiness.

The thermoplastic polymeric base material for use in the thermoplastic absorbent material of the present invention optionally may also comprise from 0% to 100%, or 1% to 30%, or from 5% to 20%, or preferably from 8% to 12% by weight of tackifying resins. As used herein, the term 'tackifying resin' means any of the thermoplastic absorbent materials described below that are useful to impart tack to the thermoplastic polymeric base material. ASTM D1878-61T defines tack as "the property of a material which enables it to form a bond of measurable strength immediately on contact with another surface". Tackifying resins may comprise resins derived from renewable resources such as rosin derivatives including wood rosin, tall oil and gum rosin as well as rosin esters, natural and synthetic terpenes and derivatives of such. Aliphatic, aromatic or mixed aliphaticaromatic petroleum based tackifiers are also useful in the invention. Representative examples of useful hydrocarbon resins can include alpha-methyl styrene resins, branched and unbranched C₅ resins, C₉ resins and C₁₀ resins, as well as styrenic and hydrogenated modifications of such. Tackifying resins range from being a liquid at 37°C to having a ring and ball softening point of about 135°C. Suitable tackifying resins for use herein may include natural and modified resins; glycerol and pentaerythritol esters of natural and modified resins; polyterpene resins; copolymers and terpolymers of natural terpenes; phenolic modified terpene resins and the hydrogenated derivatives thereof; aliphatic petroleum resins and the hydrogenated derivatives thereof; aromatic petroleum resin and the hydrogenated derivatives thereof; and aliphatic or aromatic petroleum resins and the hydrogenated derivatives thereof, and combinations thereof.

The thermoplastic polymeric base material for use in the thermoplastic absorbent material of the present invention optionally may also comprises from 0.1% to 10%, or 0.2% to 5%, more preferably from 0.5% to 2%, or preferably from 0.75% to 1.5% by weight of anti-oxidants. Suitable 'anti-oxidants' for use in the present invention may include any conventional anti-oxidants, and are suitably hindered phenols such as for example Ethanox 330™ 1,3,5-trimethyl-2,4,6-tris(3,5-di-tert-butyl-4-hydroxybenayl) benzene which is commercially available from the Ethyl Corporation. Other examples for suitable anti-oxidants are hindered phenolics (e. g., Irganox 1010, Irganox 1076, Irganox B 225).

The thermoplastic polymeric base material for use in the thermoplastic absorbent material of the present invention optionally may also comprise surfactants. Suitable 'surfactants' for use herein are additives that reduce the surface tension and/or contact angle of the thermoplastic polymeric base material. Surfactants are useful in amounts ranging from about 0 % to about 25 % by weight and desirably from about 5 % to about 15 % by weight, with respect to the total weight of the thermoplastic polymeric base material. Suitable surfactants may include nonionic, anionic, and silicone surfactants, as it is known in the art. The preferred surfactants may be those with lower molecular weights because these have increased compatibility in the thermoplastic polymeric base material. The maximum acceptable molecular weight depends on the type of surfactant and the other ingredients in the thermoplastic polymeric base material and thus the thermoplastic absorbent material of the present invention.

Other optional components of the thermoplastic polymeric base material for use herein may include anti-ultraviolets, dyes, antibacterials, odour adsorbing materials, perfumes, pharmaceuticals, and mixtures thereof, which may be present within the thermoplastic absorbent materials at a level of up to 10% by weight of the composition. it may be desirable the thermoplastic absorbent material of the present invention be a hot-melt adhesive, i.e. the thermoplastic base material can comprise a hot-melt adhesive. Such suitable thermoplastic absorbent materials comprise (by weight):
a) from about 20% to about 99% of a thermoplastic polymeric base material, comprising
   a') from about 10% to about 50% of a block copolymer,
   a") from about 0% to about 50% of a tackifying resin; and
b) from about 1% to about 80% of particles of superabsorbent material having an average particle diameter in dry state of from 0.1 µm to 500 µm.

In another embodiment the thermoplastic absorbent material of the present invention may be the following hot-melt adhesive composition, comprising (by weight):
a) from about 20% to about 99% of a thermoplastic polymeric base material, comprising
   a') from about 10% to about 50% block copolymer,
   a") from about 0% to about 50% tackifying resin,
   a"') from about 10% to about 80% plasticizer,
   a"") from about 0% to about 2% antioxidant; and
b) from about 1% to about 80% of particles of superabsorbent material having an average particle diameter in dry state of from 0.1 µm to 500 µm.

Suitable thermoplastic polymeric base materials for use in the thermoplastic absorbent materials described herein before may be those disclosed in our copending application WO 03/049777. Said thermoplastic base materials have a water absorption capacity of at least 30%, or more than 40%, or more than 60% or preferably more than 90%, when measured according to the Water Absorption Test described therein in accordance with ASTM D 570-81, on a 200 µm thick film. The intrinsic absorbency of the thermoplastic polymeric base material/matrix allows for a more effective diffusion of the body fluid within the matrix and, consequently, for a better spreading of the body fluid, which can reach a greater number of superabsorbent material particles, which in turn give rise to a better utilization of the absorbent material.

The thermoplastic base materials described in the above mentioned application WO 03/049777 provide thermoplastic absorbent materials which can be particularly suitable for the present invention having improved mechanical properties, such as good integrity in wet state thanks to good internal cohesion and hence having a tensile strength in wet state which is at least 20%, orat least 40%, or preferably at least 60% of the tensile strength of said composition in dry state. Said tensile strengths are evaluated according to the Tensile Strength Test described in WO 03/049777. It should be appreciated that by selecting a thermoplastic base material in the thermoplastic absorbent material of the present invention having a higher value of water absorption, the thermoplastic absorbent material will have better liquid absorption/handling characteristics, while not compromising on tensile strength in wet state. Indeed such thermoplastic absorbent material will remain substantially intact and have sufficient tensile strength for its intended use, also upon liquid absorption.

Indeed these thermoplastic absorbent materials for use herein offer improved mechanical and absorbent properties. Without to be bound by theory it is believed that the intrinsic absorbency of the matrix constituted by said thermoplastic base material allows the body fluid to be acquired and diffused within the matrix thus permitting fluid contact with the superabsorbent material contained in the matrix and typically their swelling, without the necessity of having a matrix of a thermoplastic base material of low cohesive strength but with a matrix which remains substantially intact and having sufficient strength upon fluid absorption.

The superabsorbent material in particle form may be blended with the thermoplastic base material in any known manner to provide the thermoplastic absorbent material of the present invention. For example, by first melting the thermoplastic polymeric base material and then by adding and mixing the required amount of superabsorbent material particles. Suitable adhesive processing equipments can be used such as a melt mixer or extruder. The thermoplastic absorbent material of the present invention may be desirably formulated to have hot melt adhesive characteristics so that they can be applied utilizing any known method used for applying hot melt adhesives. As it is known in the art, hot melt techniques are more in general desirable since they are easy to perform, without the need for complex equipment and high power for making and applying a thermoplastic material with hot melt characteristics.

At least at the coating temperature, the thermoplastic absorbent material comprising a thermoplastic polymeric base material can exhibit adhesive properties on a supportive substrate in order to form a composite structure such that no additional adhesive is required to achieve a permanent attachment between the thermoplastic absorbent material and the substrate. However, while hot melt techniques may be desirable, any other known method for processing thermoplastic compositions can be used for processing the thermoplastic absorbent materials of the present invention in any known form/pattern. Also, any known method for spraying, printing, dotting, coating or foaming thermoplastic compositions can be used as well as extrusion or lamination processes.

Particularly suitable methods for applying the thermoplastic absorbent material to a substrate may be gravure printing or slot coating. Both methods may be particularly suitable for discontinuous application of the thermoplastic absorbent material described herein onto a substrate. The gravure print unit or slot coater applies the thermoplastic absorbent material in the desired pattern onto a substrate.

### Superabsorbent material.

'Superabsorbent material' as used herein means materials, which are capable of absorbing at least five times of their weight of water or aqueous liquids. Suitable superabsorbent materials comprise anionic absorbent gelling material as well as cationic absorbent material, such as chitin, chitosan or chitosan compounds, or combinations of anionic and cationic superabsorbent material. Particularly suitable superabsorbent materials for use herein can be anionic absorbent gelling materials, i.e., absorbent gelling materials, which are predominantly negatively charged. These absorbent gelling materials can be any material having superabsorbent properties in which the functional groups are anionic, namely sulphonic groups, sulphate groups, phosphate groups or carboxyl groups. Typically the functional groups are carboxyl groups. Particularly suitable anionic absorbent gelling materials for use herein can be synthetic anionic absorbent gelling materials. Generally, the functional groups are attached to a slightly cross-linked acrylic base polymer. Superabsorbent materials for use according to the present invention can be made by polymerisation of ethylenically unsaturated monomers. Examples of ethylenically unsaturated monomers can be acrylic acid, methacrylic acid, crotonic acid, maleic acid and its anhydride, fumaric acid, itaconic acid, and 2-(meth)acryloylethanesulfonic acid, and 2-(meth)acryloylpropanesulfonic acid, and 2-(meth)acrylamido-2-methylpropanesulfonic acid, vinylsulfonic acid, styrenesulfonic acid and the like and their salts; monomers containing nonionic hydrophilic substituents such as (meth)acrylamide, N-substituted (meth)acrylamides, 2-hydroxyethyl (meth)acrylate, 2-hydroxypropyl (meth)acrylate, methoxypolyethylene glycol (meth)acrylate, polyethylene glycol (meth)acrylate and the like; monomers of cationic character such as N,N'-dimethylaminoethyl (meth)acrylate, N,N'-diethylaminoethyl (meth)acrylate, N,N',N,N'-diethylaminopropyl (meth)acrylate, N,N'-dimethylaminopropyl (meth)acrylamide, and the like and their quartary salts. The polymers of those monomers can be used alone or mixtures of the polymers two or more of those monomers can be used as well. Copolymers of these monomers can also be used. Especially suitable polymers for use as superabsorbent material can be cross-linked polyacrylates, hydrolyzed acrylonitrile grafted starch, polyacrylates grafted starch and isobutylene maleic anhydride copolymers.

Suitable crosslinking agents for facilitating the cross-linking of the preferred absorbent gelling material for use as superabsorbent material may be N,N'-methylene-bis(meth)acrylamide, N-methylol(meth)acrylamide, ethylene glycol (meth)acrylate, polyethylene glycol (meth)acrylate, propylene glycol (meth)acrylate, polypropylene glycol (meth)acrylate, glycerol tri(meth)acrylate, glycerol mono(meth)acrylate, polyfunctional metal salts of (meth)acrylic acid, trimethylolpropane tri(meth)acrylate, triallylamine, triallyl cyanulate, triallyl isocyanulate, triallyl phosphate, glycidyl (meth)acrylate. As examples of agents having reactive functional groups for example, in a case that a monomer has a carboxyl and/or carboxylate group, polyhydric alcohol derivatives such as ethylene glycol, diethylene glycol, triethylene glycol, tetraethylene glycol, polyethylene glycol, glycerol, polyglycerol, propylene glycol, diethanolamine, triethanolamine, polyoxypropylene, oxyethyleneoxypropylene block co-polymer, pentaerythritol, and sorbitol; polyglycidyl derivatives such as ethylene glycol diglycidyl ether, polyethylene glycol diglycidyl ether, glycerol polyglycidyl ether, diglycerol polyglycidyl ether, polyglycerol polyglycidyl ether, sorbitol polyglycidyl ether, pentaerythritol polyglycidyl ether, propylene glycol diglycidyl ether, and polypropylene glycol diglycidyl ether; aziridine derivatives and related compounds such as 2,2-bishydroxymethylbutanol-tris (3-[1-aziridinyl) propionate], 1,6-hexamethylene-diethylene urea, and diphenylmethane-bis-4,4'-N,N'-diethylene urea; haloepoxyl compounds such as epichlorohydrin and alpha -methylchlorohydrin; polyaldehydes such as glutar aldehyde and glyoxal; poly amine derivatives such as ethylene diamine, diethylene triamine, triethylene tetramine, tetraethylene pentamine, pentaethylene hexamine, and polyethylene hexamine; polyisocyanates such as 2,4-toluylenediisocyanate and hexamethylenediisocyanate; polyvalent metal salts such as aluminium chloride, magnesium chloride, calcium chloride, aluminium sulfate, magnesium sulfate, and calcium sulfate. Subject to consideration upon reactivity, these crosslinking agents can be used as a mixture of more than two, but it is usually preferable to use a crosslinking agent having polymerizable unsaturated groups.

The slightly cross-linked, hydrogel-forming absorbent gelling materials can generally be employed in their partially neutralized form. For purposes described herein, such materials are considered partially neutralized when at least 25 mole percent, and typically at least 50 mole percent of monomers used to form the polymer are acid group-containing monomers, which have been neutralized with a salt-forming cation. Suitable salt-forming cations may include alkali metal, ammonium, substituted ammonium and amines. This percentage of the total monomers utilized, which are neutralized acid group-containing monomers, is referred to as the "degree of neutralization". Typically, commercial absorbent gelling materials have a degree of neutralization somewhat from 25% to 90%.

Examples for cationic superabsorbent materials for use herein can be chitin, chitosan, chitosan salts, such as chitosonium lactate or chitosonium pyrrolidone carboxylate (as disclosed in WO-A-98/07618), modified chitosans as disclosed in WO-A-87/07618, US 5,378,472 or EP-A-737,692, cross-linked chitosans, or mixtures thereof.

The thermoplastic absorbent material of the present invention is capable of providing an increased absorption and retention capacity of proteinaceous or serous body fluids, for example menses, when compared to known thermoplastic absorbent materials. Its improved effectiveness can be expressed in terms of overall retention capacity towards Artificial Menstrual Fluid, evaluated according to the Centrifuge Retention Capacity Test for the thermoplastic absorbent material described herein, and can be achieved with a relatively low amount of the superabsorbent material within the thermoplastic absorbent material. More in general, according to the present invention, a thermoplastic absorbent material is provided for absorption of proteinaceous or serous body fluids, comprising a thermoplastic base material and particles of a superabsorbent material dispersed therein, which has a retention capacity towards Artificial Menstrual Fluid (AMF) of at least 9 g/g, or of at least 11 g/g, or preferably of at least 13 g/g, when said superabsorbent material is in an amount of not more than 50% by weight of said thermoplastic absorbent material. The retention capacity of the thermoplastic absorbent material is evaluated according to the Centrifuge Retention Capacity Test for the thermoplastic absorbent material described herein.

The thermoplastic absorbent materials of the present invention comprising particulate superabsorbent materials can be incorporated in absorbent articles typically in the absorbent core, in different embodiments as it is known in the art. The absorbent core can comprise the thermoplastic absorbent material in different patterns and embodiments, for example coated onto a substrate layer comprised in the core. Alternatively, the thermoplastic absorbent materials can be comprised as a continuous or discontinuous layer in layered core structures. The thermoplastic absorbent material of the present invention can also integrally constitute the absorbent core in an absorbent article, for example being applied in a suitable pattern onto a non absorbent substrate.

In certain embodiments, the thermoplastic absorbent materials of the present invention may be present in an absorbent article, typically in its absorbent core, with a basis weight of from 5 g/m² to 500 g/m², or from 10 g/m² to 100 g/m², or from 20 g/m² to 60 g/m², or preferably from 25 g/m² to 40 g/m².

The thermoplastic absorbent material of the present invention may integrally constitute the core of an absorbent article, which can in turn comprise 100% of said thermoplastic absorbent material. It may be desirable the absorbent core of an absorbent article include from 1% to 90% by weight of the thermoplastic absorbent material of the present invention, or from 5% to 50% by weight, or preferably from 10% to 30% by weight.

According to the present invention, a method is also provided for increasing the absorption of proteinaceous or serous body fluids in an absorbent article, which consists in incorporating a thermoplastic absorbent material as described herein into the absorbent article, according to the various embodiments also disclosed herein.

The invention will be illustrated with the following examples.

### Example 1

A thermoplastic absorbent material according to the present invention comprises in weight percent:

| | |
|---|---|
| 12% | Estane T5410 from Noveon Inc. |
| 30% | PEG E400 from Dow Chemical |
| 1% | Irganox B225 from Ciba Specialty Chemicals |
| 12% | CR00 (formerly PM17) from Savaré I.C. s.r.l. |
| 45% | Ground Aqualic L74 from Nippon Shokubai |

Estane T5410 is a hydrophilic thermoplastic polyurethane polymer, PEG E400 is a polyethylene glycol plasticizer (MW about 400), Irganox B225 is an antioxidant and CR00 is a commercially available hot melt adhesive. The thermoplastic base material has hot melt adhesive characteristics.

The thermoplastic absorbent material contains a superabsorbent material in particle form having irregular shape and obtained by suitably grinding the commercially available Aqualic L74 with d₉₉ = 35 µm in order to achieve an average particle size of 15 µm, and an average surface area per volume of 0.72 m²/cm³. The thermoplastic absorbent material has an Elastic Modulus G' of 450 Pa and a Tan δ of 1.3.

### Example 2

A thermoplastic absorbent material according to the present invention comprises in weight percent:

| | |
|---|---|
| 12% | Estane T5410 from Noveon Inc. |
| 30% | PEG E400 from Dow Chemical |
| 1% | Irganox B225 from Ciba Specialty Chemicals |
| 12% | CR00 (formerly PM17) from Savaré I.C. s.r.l. |
| 45% | Ground Aqualic L74 from Nippon Shokubai |

Estane T5410 is a hydrophilic thermoplastic polyurethane polymer, PEG E400 is a polyethylene glycol plasticizer (MW about 400), Irganox B225 is an antioxidant and CR00 is a commercially available hot melt adhesive. The thermoplastic base material has hot melt adhesive characteristics.

The thermoplastic absorbent material contains a superabsorbent material in particle form having irregular shape and obtained by suitably grinding the commercially available Aqualic L74 with d₉₉ = 50 µm in order to achieve an average particle size of 19 µm, and an average surface area per volume of 0.71 m²/cm³. The thermoplastic absorbent material has an Elastic Modulus G' of 350 Pa and a Tan δ of 1.72.

### Comparative Example

A thermoplastic absorbent material formulated to have hot melt adhesive characteristics comprises:

| | |
|---|---|
| 17% | Estane T5410 from Noveon Inc. |
| 18% | PEG E400 from Dow Chemical |
| 1% | Irganox B225 from Ciba Specialty Chemicals |
| 19% | CR00 (formerly PM17) from Savaré I.C. s.r.l. |
| 45% | Ground Aqualic L74 from Nippon Shokubai |

The composition contains the same ground superabsorbent material used in Example 1. However, the thermoplastic absorbent material is much harder with an Elastic Modulus G' of 280.000 Pa and a Tan δ of 0.25.

The thermoplastic absorbent materials according to the present invention and the comparative example were tested for their retention capacity towards Artificial Menstrual Fluid (AMF) according to the Centrifuge Retention Capacity Test for the thermoplastic absorbent material described herein, and the results are summarized in the table below.

| | **Example 1** | **Example 2** | **Comparative Example** |
|---|---|---|---|
| Retention Capacity for AMF | 15.1 g/g | 12.0 g/g | 4.3 g/g |
| Elastic Modulus G' @ 38°C, 1 rad⁻¹ | 450 Pa | 350 Pa | 280.000 Pa |
| Tan δ @ 38°C, 1 rad⁻¹ | 1.3 | 1.72 | 0.25 |
| Average Particle Size | 15 µm | 19 µm | 15 µm |
| Average Surface Area | 0.72 m²/cm³ | 0.71 m²/cm³ | 0.72 m²/cm³ |

The retention capacity data show the much better behaviour of the thermoplastic absorbent materials according to the present invention (Examples 1 and 2) compared to the Comparative Example. More in detail, Comparative Example comprises a superabsorbent material in particle form with irregular shape, and with low average particle size and high average surface area per volume, but in a very hard formulation with high Elastic Modulus and low Tan δ. The results show the importance of the combination of selected particle size and shape with selected rheological properties of the thermoplastic absorbent material of the present invention, which in turn provide the surprisingly excellent results in terms of retention capacity of Artificial Menstrual Fluid compared to known materials.

### Test procedures.

### Centrifuge Retention Capacity Test for the thermoplastic absorbent material.

The test is based on the Edana Recommended Test Method 441.2-02 (Centrifuge Retention Capacity), with the following changes which refer to the corresponding sections and subsections of the test method description:

### Section 1 - Scope

The scope is modified as follows: the present method determines the fluid retention capacity of a thermoplastic absorbent material comprising a thermoplastic polymeric base material and particles of a polyacrylate based material dispersed therein in Artificial Menstrual Fluid (AMF) following centrifugation.

### Section 4 - Terms and Definitions

The material for the bag is a heat sealable polyester net, as described under 7.1 below. Nonwoven, defined in 4.1 of the original test method, is not used.
- 4.2 shall read: Bag: bag of heat-sealable polyester net.

### Section 6 - Reagents

Only AMF is used, prepared as explained in the respective section of the description.

### Section 7 - Apparatus

- 7.1 The material used for the bag is a heat-sealable polyester net sold by Saatitech S.p.A. as Saatifil PES 18/13, with mesh opening 18 µm, open area 13%, mesh count 200/cm, thread diameter 31 µm. The bags are prepared as described in the original test method under this section.
- 7.2 shall read: Heat sealer, capable of bonding the heat-sealable polyester net.
- 7.3 shall read: The large pan is replaced by a beaker, of 1000 ml capacity and 100 mm inner diameter. In addition a plastic screen support is needed, for example a plastic net with square pattern and about 5 mm mesh, cut in appropriate shape with the same dimension as the polyester bag under 7.1, and with a handle e.g. constituted by a metal wire, inverted-U shaped and fixed to two opposite sides of the plastic screen support, in order to introduce the plastic screen support with the bags into the 1000 ml beaker, and to subsequently withdraw it as described in modified 9.8 below.
- 7.7 No volumetric flask is needed.

### Section 8 - Sampling

This section is replaced by the following:
The thermoplastic absorbent material is melted and then coated with known means in the form of stripes of 6 mm width and 150 µm thickness. Samples of these stripes are cut and must be kept in a closed container to avoid dust contamination, and allowed to equilibrate to the ambient laboratory temperature before preparing a test portion to run the test. The test conditions are (23 ± 2) °C and (50 ± 10) % relative humidity.

### Section 9 - Procedure

- 9.1 shall read: Bags are prepared with the net material as specified in 7.1. Each experimental test comprises four bags per test sample and two blanks.
- 9.2 shall read: Weigh, to the nearest 0.005 g, a 0.300 g test portion of absorbent material and record the mass mₛ₁.
- 9.4 shall read: Use the same procedure to prepare a second, third, and fourth sample mₛ₂, mₛ₃, mₛ₄. If it takes longer than 5 minutes to weigh and seal the bags before starting the test, place the bags in a desiccator.
- 9.6 This step is replaced by modified 9.8.
- 9.8 shall read: Lay the four bags flat, one on top of the other, on the plastic screen support. Place the plastic screen support with the bags on the bottom of the beaker, and fill the beaker with 300 ml AMF pouring it carefully onto the bags, in order to not modify the absorbent material distribution inside the bags. Change the AMF after a maximum of four bags. Eliminate entrapped air bubbles by careful manipulation of the bags.
- 9.9 shall read: After 30 ± 1 min, remove by the handle the plastic screen support with the bags from the beaker.
- 9.14 shall read: Remove the bags, weigh each and record the mass of the two blank bags m_{b1} and m_{b2}, and the mass of the bags containing the absorbent material M_{w1}, m_{w2}, m_{w3} and m_{w4}.

As also mentioned in the original test method under 9.5, the test on the blanks need not be carried out if same conditions apply.

### Section 10 - Calculation

The test is run on the four sample replicates (i = 1, 2, 3 and 4), instead of two as prescribed in the original test method, and the result is taken as the average of the four calculated values to 0.1 unit accuracy. The calculation as explained in Section 10 shall be modified accordingly.

### Thickness Measurement

The thickness of a film of the thermoplastic absorbent material of the present invention should always be measured at the thickest possible place. The thickness is measured with a micrometer gauge having a range of 0 to 30 mm and capable of ±0.5 mm tolerance. The gauge must not be spring-loaded and should have a foot moving downwards under gravity. The micrometer foot has a diameter of 40 mm and is loaded with 80 gram weight. The measurement is taken between 5 and 10 seconds after the foot has been lowered to come into contact with the absorbent article. Measurements should be taken often enough to allow statistical analysis to determine average thickness within a sigma of ±0.1 mm. A detailed description of the thickness measurement can also be found in US patent 5,009,653 .

### Water Absorption Test and Tensile Strength Test

The Water Absorption Test and the Tensile Strength Test are conducted as disclosed in PCT application WO 03/049777, assigned to The Procter & Gamble Company, at lines 11-29 of page 24, and from line 9 of page 25 to line 12 of page 26, respectively, to which reference is made.

### Average Particle Size and Average Surface Area Measurement

The average particle size and the average surface area of the superabsorbent material is measured by means of a laser diffraction analysis apparatus model Helos, with dry feeding and dispensing system Rodos, both available from Sympatec GmbH. The average particle size is measured by dry analysis on the particles in dry dispersion. The test is conducted at controlled conditions of 23°C and 50% relative humidity on the material which has been kept in such conditions for at least 24 hours before the test. The settings of the apparatus can be easily determined by the skilled man, for example the average particle size and average surface area of the particulate material of the Examples described herein has been measured with the following settings specific for the Helos apparatus: measuring field R3; focus length 100 mm; measurement field 0.5-175 µm; optical concentration 2-15%.

Alternatively, the average particle size and average surface area could be also measured in a liquid suspension, for example suspending at room temperature the particulate superabsorbent material into a suitable solvent which does not interact with the particulate material, and analyzing the liquid suspension for example with the Helos analyzer provided with a suitable wet dispersing system also available from Sympatec GmbH. This alternative procedure can be useful for particulate superabsorbent materials obtained from a thermoplastic absorbent material, as explained below.

### Alternative sample preparation for all tests herein when starting from an absorbent article.

When starting from an article comprising the thermoplastic absorbent material, for example a disposable absorbent article with the thermoplastic absorbent material coated onto a substrate, the thermoplastic absorbent material can be isolated with known means in order to be tested. Typically, in a disposable absorbent article the topsheet is removed from the backsheet and both are separated from any additional layers if present. The thermoplastic absorbent material is removed from its substrate layer, e.g. by scraping with a spatula. The recovered thermoplastic absorbent material will be used to prepare samples as mentioned above with known means. For example, the thermoplastic absorbent material can be melted, or dissolved with a suitable solvent.

Particles of superabsorbent material can be also separated from the thermoplastic absorbent material, in order to isolate and separate the thermoplastic base material and the particles of superabsorbent material which shall be used to prepare samples, as it is known in the art, for example by suitably sieving or filtering from the molten state, or preferably from the solution, e.g. by washing with a suitable solvent which does not interact with the superabsorbent material, as can be readily determined by the man skilled in the art.

### Artificial Menstrual Fluid (AMF)

Artificial Menstrual Fluid is based on modified sheep's blood that has been modified to ensure it closely resembles human menstrual fluid in viscosity, electrical conductivity, surface tension and appearance. It is prepared as explained in US Patent 6,417,424, assigned to The Procter & Gamble Company, from line 33 of column 17 to line 45 of column 18, to which reference is made.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A thermoplastic absorbent material comprising a thermoplastic base material and particles of a superabsorbent material dispersed therein, wherein said particles have an irregular shape and an average particle size in dry state of from 0.1 µm and 50 µm, preferably from 1 µm and 30 µm, more preferably from 1 µm and 20 µm, and wherein said thermoplastic absorbent material has an Elastic Modulus G' of from 50 Pa to 100.000 Pa, preferably from 75 and 50.000 Pa, more preferably from 75 and 10.000 Pa, most preferably from 100 and 1.000 Pa, measured at 38°C and 1 rad⁻¹.

2. A thermoplastic absorbent material according to claim 1, wherein said particles have an average surface area per volume of at least 0.50 m²/cm³, preferably at least 0.60 m²/cm³, more preferably at least 0.70 m²/cm³.

3. A thermoplastic absorbent material according to any preceding claim, wherein said thermoplastic absorbent material has a Tan δ of at least 0.15, preferably at least 0.30, and more preferably at least 0.45, measured at 38°C and 1 rad⁻¹.

4. A thermoplastic absorbent material according to any preceding claim, wherein said thermoplastic absorbent material comprises from 20% to 99%, preferably from 30% to 90%, most preferably from 40% to 70% by weight of the total composition of a thermoplastic polymeric base material and from 1% to 80%, preferably from 10% to 70%, and most preferably from 30% to 60%, by weight of the total composition of particles of superabsorbent material.

5. A thermoplastic absorbent material according to any preceding claim, wherein said thermoplastic polymeric base material comprises a thermoplastic polymer or mixture of thermoplastic polymers, and a suitable compatible plasticiser or a blend of suitable compatible plasticisers.

6. A thermoplastic absorbent material according to any preceding claim, wherein said thermoplastic polymeric base material is a hot melt adhesive.

7. A thermoplastic absorbent material according to any of claims 4 to 6, wherein said thermoplastic polymeric base material comprises from 5% to 99%, preferably from 10% to 90%, more preferably from 30% to 70%, most preferably from 40% to 60% by weight of said thermoplastic polymer or mixture of polymers, and from 5% to 90%, preferably from 10% to 85%, more preferably from 15% to 70%, most preferably from 30% to 65% by weight of said suitable compatible plasticiser or blend of suitable compatible plasticisers.

8. A thermoplastic absorbent material according to any of claims 4 to 7, wherein said thermoplastic polymeric base material has a water absorption greater than 30%, preferably greater than 40%, more preferably greater than 60%, most preferably greater than 90%.

9. An absorbent article for absorption of proteinaceous or serous body fluids, comprising a thermoplastic absorbent material according to any preceding claim.

10. A method for increasing the absorption of proteinaceous or serous body fluids in an absorbent article by incorporating in said absorbent article a thermoplastic absorbent material according to any of claims 1 to 9.
